(19) Europäisches Patentamt
European Patent Office
Office européen des brevets

(11) Numéro de publication: **0 384 844 A2**

(12) **DEMANDE DE BREVET EUROPEEN**

(21) Numéro de dépôt: **90400495.9**

(22) Date de dépôt: **22.02.90**

(51) Int. Cl.5: **C07D 211/96, C07D 207/48, A61K 31/44, A61K 31/40**

(30) Priorité: **23.02.89 IT 1954389**

(43) Date de publication de la demande: **29.08.90 Bulletin 90/35**

(84) Etats contractants désignés: **AT BE CH DE ES FR GB GR IT LI LU NL SE**

(71) Demandeur: **ROUSSEL-UCLAF**
**35, boulevard des Invalides**
**F-75007 Paris(FR)**

(72) Inventeur: **Toja, Emilio**
**Via Plezzo 80**
**Milano(IT)**
Inventeur: **Barzaghi, Fernando**
**Via Monteverdi 21**
**F-20052 Monza, Milan(IT)**
Inventeur: **Galliani, Giulio**
**13, Via Silva**
**1 Monza(IT)**

(74) Mandataire: **Tonnellier, Marie-José et al**
**111, route de Noisy B.P. no 9**
**F-93230 Romainville(FR)**

(54) **Nouveaux dérivés de la 1-arylsulfonyl pyrrolidin-2-thione ou de la 1-arylsulfonyl pipéridin-2-thione, leur procédé de préparation, leur application comme médicaments et les compositions les renfermant.**

(57) L'invention concerne les produits de formule

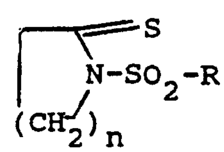

(I)

dans laquelle
- ou bien R représente

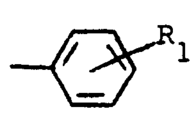

où $R_1$ représente soit un alcoyle ($C_{1-8}$), soit

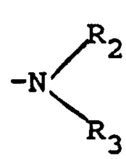

EP 0 384 844 A2

Xerox Copy Centre

$R_2$ et $R_3$ représentant H, alcoyle ($C_{1-8}$) ou avec l'azote forment un hétérocyclique renfermant éventuellement un autre hétéroatome, soit $OR'$, $R'$ étant H, alcoyle ($C_{1-8}$) ou aryle (--> $C_{14}$), soit $SR_4$ ou $S(O)R_5$, $R_4$ et $R_5$ étant alcoyle ($C_{1-8}$),
- ou bien naphtyle, éventuellement substitué par $R'_1$, $R'_1$ pouvant avoir les valeurs de $R_1$ et n représente 1 ou 2, leur procédé de préparation, leur application comme médicaments et les compositions les renfermant.

**Nouveaux dérivés de la 1-arylsulfonyl pyrrolidin-2-thione ou de la 1-arylsulfonyl pipéridin-2-thione, leur procédé de préparation, leur application comme médicaments et les compositions les renfermant.**

La présente invention concerne de nouveaux dérivés de la 1-arylsulfonyl pyrrolidin-2-thione ou de la 1-arylsulfonyl pipéridin-2-thione, leur procédé de préparation et leur aplication comme médicaments et les compositions les renfermant.

L'invention a pour objet les composés de formule (I) :

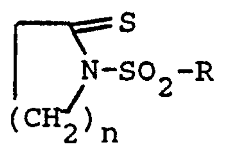

(I)

dans laquelle

- ou bien R représente un radical

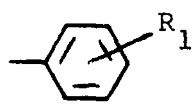

dans laquel $R_1$ en position quelconque sur le noyau phényle représente

soit un radical alcoyle linéaire, ramifié ou cyclique, saturé ou insaturé renfermant jusqu'à 8 atomes de carbone,

soit un radical

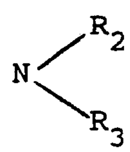

dans lequel $R_2$ et $R_3$ identiques ou différents représentent un atome d'hydrogène, un radical alcoyle linéaire, saturé ou insaturé renfermant jusqu'à 8 atomes de carbone, ou forment ensemble avec l'atome d'azote auquel ils sont liés un radical hétérocyclique carboné renfermant éventuellement un autre hétéroatome,

soit un radical $OR'$, $R'$ représentant un atome d'hydrogène, un radical alcoyle linéaire, ramifié ou cyclique renfermant jusqu'à 8 atomes de carbone, ou un radical aryle renfermant jusqu'à 14 atomes de carbone,

soit un radical $SR_4$ ou $S(O)R_5$, $R_4$ et $R_5$ représentant un radi cal alcoyle linéaire, ramifié ou cyclique, saturé ou insaturé renfermant jusqu'à 8 atomes de carbone,

- ou bien R représente un radical naphtyle, éventuellement substitué par un radical $R'_1$, $R'_1$ pouvant prendre l'une des valeurs indiquées ci-dessus pour $R_1$ et n représente le nombre 1 ou le nombre 2.

Par radical alcoyle, on entend de préférence un radical alcoyle renfermant de 1 à 6 atomes de carbone, par exemple le radical méthyle, éthyle, n-propyle, isopropyle, n-butyle, isobutyle, tert-butyle, n-pentyle, n-hexyle, cyclopropyle, cyclobutyle, cyclopentyle ou cyclohexyle.

Par radical alcoyle insaturé, on entend de préférence un radical éthényle, propényle ou butényle.

Lorsque $R_2$ et $R_3$ forment avec l'atome d'azote auquel ils sont liés un radical hétérocyclique renfermant éventuellement un autre hétéroatome, il s'agit de préférence d'un radical piperidyle, piperazinyle, morpholinyle, pyrrolidinyle ou hexahydroazépinyle.

Parmi les composés préférés de l'invention, on peut citer les composés dans lesquels R représente un radical

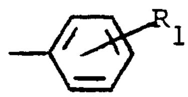

$R_1$ conservant la même signification que précédemment, ceux dans lesquels le radical $R_1$ est en position 4, ceux dans lesquels $R_1$ représente un radical alcoyle linéaire ou ramifié renfermant jusqu'à 8 atomes de carbone et notamment un radical tert-butyle, ceux dans lesquels $R_1$ représente un radical

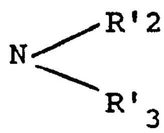

dans lequel $R'_2$ et $R'_3$ représentent un radical alcoyle linéaire ou ramifié renfermant jusqu'à 8 atomes de carbone ou forment avec l'atome d'azote auquel ils sont liés un radical hétérocyclique ou encore ceux dans lesquels $R_1$ représente un radical $SR_4$, $R_4$ représentant un radical alcoyle linéaire ou ramifié renfermant jusqu'à 4 atomes de carbone et notamment un radical méthyle.

L'invention a plus particulièrement pour objet les produits dont la préparation est donnée ci-après dans la partie expérimentale à savoir les produits des exemples 1 à 6.

Les composés de formule (I) présentent d'intéressantes propriétés pharmacologiques et notamment une activité antimuscarinique spécifique et sélective.

L'invention a donc pour objet les produits de formule (I) en tant que médicaments, utiles notamment pour le traitement des troubles spasmodiques divers en gastro-entérologie, en gynécologie, en obstétrique, en urologie, en hépatologie et en radiologie.

L'invention a plus particulièrement pour objet en tant que médicament, les composés préférés cités ci-dessus, à savoir les produits des exemples 1 à 6.

La posologie usuelle est variable selon l'affection en cause, le sujet traité et la voie d'administration, elle peut être comprise entre 10 mg et 1 g par jour, par exemple entre 30 et 60 mg par jour en une ou plusieurs prises pour le produit de l'exemple 1 administré par voie orale.

La présente invention a également pour objet les compositions pharmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ;
elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), caractérisé en ce que l'on soumet un composé de formule (II) :

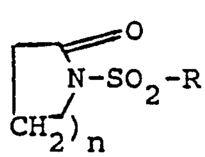

(II)

dans laquelle n et R conservent leur signification précédente à l'action d'un agent capable de convertir le radical carbonyle en radical thiocarbonyle pour obtenir le composé de formule (I) correspondant.

Comme agent capable de convertir le radical carbonyle en radical thiocarbonyle, on peut citer le réactif de LAWESSON de formule :

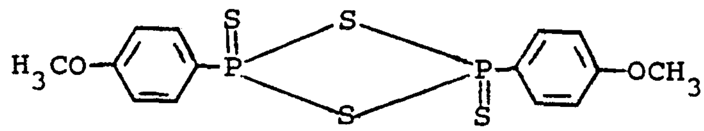

<(cf. TETRAHEDRON LETTERS 41, 2567 (1985) ou TETRAHEDRON LETTERS 41, 5061 (1985)>.
On peut également utiliser d'autres réactifs de thionation comme $P_2S_5$, $P_2S_5$-pipéridine, $P_2S_5$-TEA, $P_2S_5$-$NaHCO_3$, $PCl_5$-$Alc_2S_3$-$Na_2SO_4$.

Les produits de formule (II), dans lesquels n représente le nombre 1, sont décrits et revendiqués dans la demande de brevet européen publiée sous le n° 0 033 578, et intitulée "Nouveaux dérivés de la 1-aryl 2-pyrrolidinone, leur procédé de préparation et les nouveaux intermédiaires ainsi obtenus, leur application comme médicaments et les compositions les renfermant."

Les composés de formule (II), dans lesquels n représente le nombre 2, sont décrits et revendiqués dans la demande de brevet italien n° 19543-A/89 déposée par la Société demanderesse intitulée : "Nouveaux dérivés de la 1-arylsulfonyl 2-pipéridone, leur procédé de préparation et leur application comme médicaments."

Ils peuvent être préparés selon un procédé, caractérisé en ce que l'on soumet un composé de formule ($II_A$) :

R-$SO_2$-Hal    ($II_A$)

dans laquelle Hal représente un atome de chlore ou de brome et R conserve la signification indiquée précédemment, à l'action de l'acide 5-amino valérique ($III_A$) :

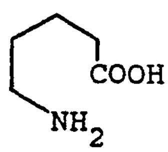

($III_A$)

pour obtenir un composé de formule ($IV_A$) :

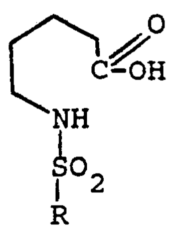

($IV_A$)

que l'on cyclise pour obtenir le produit de formule (II) correspondant dans lequel n représente le nombre 2.

Dans un mode de réalisation préférée du procédé de l'invention :

- La condensation du composé ($II_A$) avec l'acide 5-amino valérique est réalisée dans les conditions classiques de SCHOTTEN-BAUMANN, en présence d'une base comme la soude ou la potasse par exemple au sein d'un solvant organique comme le tétrahydrofuranne.
- La cyclisation du composé de formule ($IV_A$) est réalisée au moyen d'un anhydride d'acide, comme l'anhydride acétique ou encore au moyen d'autres agents de condensation comme l'acide sulfurique, l'anhydride phosphorique, l'acide phosphorique, l'acide métaphosphorique, le dicyclohexylcarbodiimide en présence de pyridine ou la bis-(triméthylsilyl) amine avec le chlorure de triméthylsilyle.

Les composés de formule ($II_A$) utilisés comme produits de départ sont connus de façon générale [cf. Houben Weyl, 4ème éd., vol. 9, Ch 18 (1955)].

Les exemples suivants illustrent l'invention sans toutefois la limiter.

**Exemple 1:1-[4-(diéthylamino) benzènesulfonyl] pyrrolidin-2-thione.**

On chauffe 1 heure au reflux un mélange comprenant 9 g de [1-(4-diéthylamino benzène sulfonyl) 2-pyrrolidinone préparé comme à l'exemple 2 du brevet européen publié sous le n° 0 033578 et 6,15 g de réactif de Lawesson (ou 2,4-bis-(4-méthoxyphényl) 1,3,2,4-dithio diphosphoéthane 2,4-disulfure) dans 180 cm³ de toluène. On laisse revenir à température ambiante, évapore à sec, reprend le résidu dans 200 cm³ de chloroforme, sèche, filtre sur célite, et évapore à sec. On récupère 3,6 g de produit brut (F = 170° 180° C) que l'on chromatographie sur silice (éluant : acétate d'éthyle-n-hexane 2-1) puis cristallise le résidu dans l'éthanol. On obtient 2,4 g de produit attendu. F = 183-184° C.

| Analyse : $C_{14}H_{20}N_2O_2S_2$ | | | |
|---|---|---|---|
| Calculé :<br>Trouvé : | C% 53,81<br>54,03 | H% 6,45<br>6,38 | N% 8,97<br>9,02 |

**Exemple 2:1-[4-(diéthylamino) benzènesulfonyl] pipéridin-2-thione.**

On chauffe 1 heure au reflux une suspension comprenant 5,5 g de 1-[4-(diéthylamino) benzylsulfonyl] 2-pipéridinone et 3,74 g de réactif de Lawesson dans 80 cm³ d'éther diméthylique d'éthylène glycol. On laisse revenir à température ambiante puis évapore à sec. On chromatographie le résidu sur silice (éluant : acétate d'éthyle-n-hexane 1-1) et obtient 4 g de produit (F = 135-140° C) que l'on purifie par cristallisation dans l'éthanol à 3 reprises. On obtient 2 g de produit pur attendu.

| Analyse : $C_{15}H_{22}N_2O_2S_2$ | | | |
|---|---|---|---|
| Calculé :<br>Trouvé : | C% 55,18<br>55,32 | H% 6,79<br>6,77 | N% 8,58<br>8,73 |

**La 1-[4-(diéthylamino) benzènesulfonyl] 2-pyrrolidinone** utilisée au départ de l'exemple 2 a été préparé comme suit :

**Stade A :**

Acide 5-(4-diéthylaminobenzènesulfonyl) valérique.

Dans une solution comprenant 1,17 g d'acide 5-amino valérique et 1,2 g de soude en solution dans 12 cm³ d'eau, on ajoute 2,5 g de chlorure de 4-diéthylamino benzène sulfonyle puis 12 cm³ de tétrahydrofuranne afin d'obtenir une solution. On maintient 2 heures sous agitation à température ambiante, évapore le tétrahydrofuranne, acidifie le milieu réactionnel à l'aide d'acide acétique, extrait au chloroforme, sèche la phase organique et élimine les solvants sous pression réduite. On obtient 2,2 g de produit attendu. F = 106-108° C. Après cristallisation dans le mélange isopropanol-eau (1-1), on obtient le produit fondant à 109-110° C.

| Analyse : $C_{15}H_{24}N_2O_4S$ | | | |
|---|---|---|---|
| Calculé :<br>Trouvé : | C% 54,86<br>54,63 | H% 7,36<br>7,28 | N% 8,53<br>8,65 |

**Stade B :**

1-[4-(diéthylamino) benzènesulfonyl] 2-pipéridinone.

On chauffe 2 heures au reflux 2 g de produit obtenu au stade A avec 2 g d'acétate de sodium dans 20 $cm^3$ d'anhydride acétique. On refroidit à température ambiante, évapore à sec, reprend le résidu dans un mélange de chloroforme et d'eau, sépare la phase organique, la sèche et obtient après élimination des solvants 1,8 g de produit brut attendu. F = 118-120° C. Après cristallisation dans l'isopropanol, on obtient 1,2 g de produit pur. F = 122-123° C.

| Analyse : $C_{15}H_{22}N_2O_3S$ | | | |
|---|---|---|---|
| Calculé : | C% 58,04 | H% 7,14 | N% 9,02 |
| Trouvé : | 58,28 | 7,34 | 9,09 |

Le chlorure de 4-(diéthylamino) benzènesulfonyle utilisé comme produit de départ a été préparé comme indiqué dans la demande de brevet européen publié sous le n° 0 033 578.

**Exmple 3 : 1-[4-(1-pipéridinyl) benzènesulfonyl] pyrrolidin-2-thione.**

1) On chauffe 1 heure au reflux un mélange comprenant 5 g de 1-[4-(1-pipéridinyl) benzènesulfonyl] 2-pyrrolidinone préparée comme à l'exemple 10 du brevet européen publié sous le n° 0 033 578 et 3,28 g de réactif de Lawesson (ou 2,4-bis-(4-méthoxyphényl) 1,3,2,4-dithio diphosphoéthane 2,4-disulfure) dans 100 $cm^3$ de diméthoxyéthane. On laisse revenir à température ambiante, évapore à sec, et chromatographie le résidu sur silice (éluant : acétate d'éthyle-n-hexane 1-1). On récupère 3,5 g de produit. F = 195-200° C. Après cristallisation dans l'éthanol, on obtient 1,5 g de produit attendu. F = 211-212° C.

| Analyse : $C_{15}H_{20}N_2O_2S_2$ | | | |
|---|---|---|---|
| Calculé : | C% 55,53 | H% 6,21 | N% 8,63 |
| Trouvé : | 55,34 | 6,23 | 8,48 |

2) On chauffe 48 heures au reflux un mélange comprenant 9,1 g de 1-[4-(1-pipéridinyl) benzènesulfonyl] 2-pyrrolidinone préparé comme à l'exemple 10 du brevet européen publié sous le n° 0 033 578 et 5,96 g de réactif de Lawesson (ou 2,4-bis (4-méthoxyphényl) 1,3,2,4-dithio diphosphoéthane 2,4-disulfure) dans 180 $cm^3$ de tétrahydrofuranne. On laisse revenir à température ambiante, évapore à sec, et chromatographie le résidu sur silice (éluant : benzène). On récupère 4,6 g de produit. F = 195-200° C. Après cristallisation dans le mélange chloroforme-n-hexane, on obtient 4,4 g de produit attendu. F = 209-211° C.

| Analyse : $C_{15}H_{20}N_2O_2S_2$ | | | |
|---|---|---|---|
| Calculé : | C% 55,53 | H% 6,21 | N% 8,63 |
| Trouvé : | 55,21 | 6,25 | 8,54 |

**Exemple 4 : 1-[4-(1-pipéridinyl) benzènesulfonyl] pipéridin-2-thione.**

On chauffe 1 heure au reflux une suspension comprenant 6 g de 1-[4-(1-pipéridinyl) benzènesulfonyl] 2-pipéridinone et 3,76 g de réactif de Lawesson dans 120 $cm^3$ de diméthoxyéthane. On laisse revenir à température ambiante puis évapore à sec. On chromatographie le résidu sur silice (éluant : acétate d'éthyle-n-hexane 1-1) et obtient 4 g de produit (F = 140-145° C) que l'on purifie par cristallisation dans le méthanol à 3 reprises. On obtient 1,5 g de produit pur attendu. F = 155-156° C.

| Analyse : $C_{16}H_{22}N_2O_2S_2$ | | | |
|---|---|---|---|
| Calculé : | C% 56,77 | H% 6,55 | N% 8,28 |
| Trouvé : | 56,54 | 6,51 | 8,43 |

**La 1-[4-(1-pipéridinyl) benzènesulfonyl] 2-pipéridinone** utilisée au départ de l'exemple 4 a été préparée comme suit :

**Stade A :**

Chlorure de 4-pipéridinylamino benzènesulfonyle.

A une solution comprenant 8,46 g d'anhydride sulfurique dans 45 $cm^3$ de chlorure de méthylène refroidie entre 0° C et -5° C, on ajoute goutte à goutte 9,3 g de dioxane puis 17,1 g de N-phényl pipéridine dissous dans 45 $cm^3$ de chlorure de méthylène. On laisse revenir à température ambiante, puis chauffe au reflux pendant 1 heure, évapore à sec, neutralise avec une solution de carbonate de sodium à 10%, concentre la phase aqueuse et sèche le résidu que l'on traite par 200 $cm^3$ de chlorure de phosphoryle et 21,8 g de pentachlorure de phosphore pendant 12 heures à température ambiante. On évapore à sec, reprend le résidu au chloroforme avec un peu d'eau, sépare la phase organique, la sèche sur sulfate de sodium, filtre et évapore le solvant. On obtient 19 g de produit attendu que l'on utilise tel quel au stade suivant.

**Stade B :**

Acide 5-(4-1-hexahydroazépinyl benzènesulfonylamino) valérique.

Dans une solution comprenant 3,51 g d'acide 5-amino valérique et 3,6 g de soude en solution dans 35 $cm^3$ d'eau, on ajoute 7,8 g de chlorure de 4-pipéridine benzène sulfonyle puis 35 $cm^3$ de tétrahydrofuranne afin d'obtenir une solution. La température s'élève à 35° C. On maintient 4 heures sous agitation à température ambiante, évapore le tétrahydrofuranne, acidifie le milieu réactionnel à l'aide d'acide acétique, dilue avec 100 $cm^3$ d'eau, extrait au chloroforme, sèche la phase organique et élimine les solvants sous pression réduite. On obtient 5,8 g de produit attendu. F = 115-120° C. Après cristallisation dans le mélange isopropanol-eau (1-1), on obtient le produit fondant à 120-122° C.

| Analyse : $C_{16}H_{24}N_2O_4S$ | | | |
|---|---|---|---|
| Calculé : | C% 56,45 | H% 7,10 | N% 8,23 |
| Trouvé : | 56,19 | 7,05 | 8,06 |

**Stade C :**

1-[4-(1-pipéridinyl) benzènesulfonyl] 2-pipéridinone.

On chauffe 4 heures au reflux 5 g de produit obtenu au stade B avec 5 g d'acétate de sodium dans 50 $cm^3$ d'anhydride acétique. On refroidit à température ambiante, évapore à sec, reprend le résidu dans 50 cm3 d'eau, filtre, sèche et obtient 4,4 g de produit brut attendu. F = 140-145° C. Après cristallisation dans l'éthanol, on obtient 3,4 g de produit pur. F = 145-146° C.

| Analyse : $C_{16}H_{22}N_2O_3S$ | | | |
|---|---|---|---|
| Calculé :<br>Trouvé : | C% 59,60<br>59,51 | H% 6,88<br>6,97 | N% 8,69<br>8,63 |

**Exemple 5 : 1-[4-(1-hexahydroazépinyl) benzènesulfonyl] pyrrolidin-2-thione.**

1) On chauffe 3 heures au reflux un mélange comprenant 5 g de 1-[4-(1-hexahydroazépinyl) benzènesulfonyl] 2-pyrrolidinone préparé comme à l'exemple 18 de la demande de brevet européen publié sous le n° 0 033 578 et 3,13 g de réactif de Lawesson (ou 2,4-bis-(4-méthoxyphényl) 1,3,2,4-dithio diphosphoéthane 2,4-disulfure) dans 100 $cm^3$ de diméthoxyéthane. On laisse revenir à température ambiante, évapore à sec, et chromatographie le résidu sur silice (éluant : benzène). On récupère 2 g de produit. F = 168-170° C. Après cristallisation dans le méthanol, on obtient 1,5 g de produit attendu. F = 172-173° C.

| Analyse : $C_{16}H_{22}N_2O_2S_2$ | | | | |
|---|---|---|---|---|
| Calculé :<br>Trouvé : | C% 56,77<br>56,85 | H% 6,55<br>6,57 | N% 8,28<br>8,23 | S% 18,95<br>18,86 |

2) On chauffe 48 heures au reflux un mélange comprenant 12,53 g de 1-[4-(1-hexahydroazépinyl) benzènesulfonyl] 2-pyrrolidinone préparé comme à l'exemple 18 du brevet européen publié sous le n° 0 033 578 et 7,5 g de réactif de Lawesson (ou 2,4-bis-(4-méthoxyphényl) 1,3,2,4-dithio diphosphoéthane 2,4-disulfure) dans 250 $cm^3$ de tétrahydrofuranne. On laisse revenir à température ambiante, évapore à sec, et chromatographie le résidu sur silice (éluant : benzène). On récupère 7,3 g de produit. F = 173-175° C. Après cristallisation dans le mélange chloroforme-n-hexane, on obtient 6 g de produit pur attendu. F = 176-178° C.

| Analyse : $C_{16}H_{22}N_2O_2S_2$ | | | |
|---|---|---|---|
| Calculé :<br>Trouvé : | C% 56,77<br>56,63 | H% 6,55<br>6,55 | N% 8,28<br>8,20 |

**Exemple 6 : 1-[4 (1-hexahydroazépinyl) benzènesulfonyl] pipéridin-2-thione.**

On chauffe 3 heures au reflux une solution comprenant 5 g de 1-[4-(1-hexahydroazépinyl) benzènesulfonyl] 2-pipéridinone et 3 g de réactif de Lawesson dans 100 $cm^3$ de 1,2-diméthoxyéthane. On laisse revenir à température ambiante, évapore à sec. On chromatographie le résidu sur silice (éluant : benzène) et obtient 2,5 g de produit (F = 146-150° C) que l'on cristallise dans l'éthanol à 2 reprises puis dans le méthanol. On obtient 1,5 g de produit pur attendu. F = 152-153° C.

| Analyse : $C_{17}H_{24}N_2O_2S_2$ | | | |
|---|---|---|---|
| Calculé :<br>Trouvé : | C% 57,92<br>57,84 | H% 6,86<br>6,87 | N% 7,95<br>7,79 |

La **1-[4-(1-hexahvdroazépinyl) benzylsulfonyl] 2-pipéridinone** utilisée au départ de l'exemple 6 a été préparée comme suit :

**Stade A :**

Chlorure de 4-(hexahydroazépinyl) benzènesulfonyle.

A une solution comprenant 2,64 g d'anhydride sulfurique dans 78 $cm^3$ de chlorure de méthylène refroidie entre +5° C et +10° C, on ajoute goutte à goutte 2,91 g de dioxane puis 5,26 g de 1-phényl hexahydroazépine (Tetrahedron 41 (1985) p. 101-106) dans 53 $cm^3$ de chlorure de méthylène. On laisse revenir à température ambiante, puis chauffe au reflux pendant 2 heures, refroidit de nouveau à température ambiante, ajoute 200 $cm^3$ d'éther éthylique à la suspension, filtre le précipité, le lave à l'éther, le sèche et obtient 7,2 g d'acide (F = 235° C décomp) que l'on traite par 36 $cm^3$ de chlorure de phosphoryle dans 36 $cm^3$ de chlorure de méthylène et par 5,87 g de pentachlorure de phosphore pendant 4 heures à température ambiante. On évapore à sec, reprend le résidu avec 100 $cm^3$ d'eau et 150 $cm^3$ de chloroforme, sépare la phase organique, la sèche sur sulfate de sodium, filtre et évapore le solvant. On obtient 6,6 g de produit attendu. F = 85-88° C.

**Stade B :**

Acide 5-(4-1-hexahydroazépinyl benzènesulfonylamino) valérique.

Dans une solution comprenant 2,56 g d'acide 5-amino valérique et 2,63 g de soude en solution dans 60 $cm^3$ d'eau, on ajoute 6 g de chlorure de 4-hexahydroazépine benzène sulfonyle puis 60 $cm^3$ de tétrahydrofuranne afin d'obtenir une solution. On maintient 2 heures sous agitation à température ambiante, évapore le tétrahydrofuranne, acidifie le milieu réactionnel à l'aide d'acide acétique, filtre le précipité, le lave à l'eau, le sèche et obtient 4,65 g de produit attendu. F = 135-140° C. Après cristallisation dans l'isopropanol, on obtient le produit fondant à 139-140° C.

| Analyse : $C_{17}H_{26}N_2O_4S$ | | | |
|---|---|---|---|
| Calculé : | C% 57,60 | H% 7,39 | N% 7,90 |
| Trouvé : | 57,46 | 7,37 | 7,98 |

**Stade C :**

1-[4-(1-hexahydroazépinyl) benzènesulfonyl] 2-pipéridinone.

On chauffe 1 heure au reflux 4 g de produit obtenu au stade B avec 4 g d'acétate de sodium dans 80 $cm^3$ d'anhydride acétique. On refroidit à température ambiante, évapore à sec, reprend le résidu dans 60 $cm^3$ d'eau, filtre, sèche et obtient 3,5 g de produit attendu. Après cristallisation dans l'isopropanol, on obtient 2,3 g de produit. F = 164-165° C.

| Analyse : $C_{17}H_{24}N_2O_3S$ | | | |
|---|---|---|---|
| Calculé : | C% 60,69 | H% 7,19 | N% 8,33 |
| Trouvé : | 60,75 | 7,09 | 8,41 |

**Exemples de compositions pharmaceutiques.**

a) On a préparé des comprimés répondant à la formule suivante :

| | |
|---|---|
| - Produit de l'exemple 1 | 10 mg |
| - Excipient q.s. pour un comprimé terminé à | 300 mg |
| (Détail de l'excipient : lactose, amidon de blé, amidon traité, amidon de riz, stéarate de magnésium, talc). | |

b) On a préparé des gélules répondant à la formule suivante :

| | |
|---|---|
| - Produit de l'exemple 1 | 20 mg |
| - Excipient q.s. pour une gélule termineé à | 300 mg |
| (Excipient : talc, stéarate de magnésium, aérosil). | |

## ETUDES BIOCHIMIQUES ET PHARMACOLOGIQUES

1) **Liaison à des différents récepteurs cérébraux.**

a) Récepteur muscarinique 1.

Sa préparation est faite à partir de Cortex prélevés sur des cerveaux de rats mâles pesant 150 à 200 g, (Iffa Crédo) broyé au Polytron dans un tampon Na/K 10 mM pH 7,4. Après incubation (aliquotes de 0,5 ml d'homogénat) pendant 60 minutes à 25° C en présence de 0,25 nM de $^3$H pirenzépine soit seule, soit avec le produit à tester, soit avec un excès de pirenzépine à $10^{-5}$M (pour déterminer la radioactivité fixée non spécifique), les incubats sont refroidis et filtrés.

La filtration est effectuée sur filtres Whatman GF/C prélavés dans une solution de polyéthylène imine à 0,05%. Les filtres sont rincés par 3 x 5 ml de tampon phosphate Na/K 10 mM pH 7,4 puis on effectue les mesures par scintillation liquide.

b) Récepteur muscarinique 2.

La préparation est effectuée à partir de cerveaux de rats mâles pesant 150 à 200 g (Iffa Crédo).

Les cerveaux sont broyés (Téflon-verre) dans une solution de sucrose 0,32 M. L'homogénat est centrifugé 10 minutes à 1000 g (0 - 4° C).

Le surnageant obtenu est recueilli et centrifugé à 30000 g pendant 15 minutes (0 - 4° C).

Le culot est remis en suspension dans un tampon Tris 50 mM pH 7,5 et le nouvel homogénat est centrifugé de nouveau à 30 000 g pendant 15 minutes (0 - 4° C).

Les culots, après élimination du surnageant, peuvent être utilisés aussitôt ou conservés jusqu'à 1 mois à -30° C.

Pour une expérience les culots sont d'abord décongelés, si nécessaire, à température ambiante et remis en suspension à l'aide d'un Dounce dans du tampon Tris 50 mM pH 7,5. Des aliquotes de 2 ml sont mis à incuber 60 minutes à 25° C en présence de 0,3 nM de $^3$H quinuclidinyl benzylate soit seul, soit avec le produit à tester, soit avec de la benzatropine à $10^{-5}$M pour déterminer la radioactivité fixée non spécifique.

A la fin de l'incubation, les tubes d'incubats sont refroidis à 4° C et filtrés rapidement sur filtres Whatman GF/C. Les filtres sont rincés par 3 x 5 ml de tampon Tris 50 mM pH 7,5 puis on effectue les mesures par scintillation liquide (Henry I Yamamura, Solomon H. Snyder, Proc. Nat. Acad. Sc. (1974) 71, n° 5, 1725-1729).

Les résultats sont exprimés en $CI_{50}$ (concentration nécessaire pour inhiber de 50% la radioactivité

spécifique fixée).

TABLEAU 1

| Composé de l'exemple | Affinité pour les récepteurs muscariniques $M_1$ et $M_2$ | |
|---|---|---|
| | [3H]pirenzépine | [3H]quinuclidinyl benzylate |
| 1 | 43 | 2200 |
| 2 | 73 | 1900 |
| 3 | 25 | 650 |
| 4 | 30 | 580 |
| 5 | 10 | 120 |
| 6 | 12 | 230 |

Les composés des exemples 1 à 6 montrent une remarquable et intéressante affinité pour le récepteur muscarinique, et principalement pour le récepteur de type $M_1$. Par contre les mêmes composés ont montré une affinité négligeable ($CI_{50}$ > 5000-10000) pour les autres récepteurs examinés parmi lesquels ont peut citer ceux de la dopamine, de l'histamine, de la sérotonine (5 $HT_1$ et 5 $HT_2$), des benzodiazépines, du GABA, des adrénorécepteurs (alpha1, alpha2, béta1, béta2) ou encore les récepteurs opiacés (mu, kappa).

2) **Intéraction et affinité avec différents récepteurs intestinaux.**

L'intéraction des composés avec différents récepteurs a été évaluée sur l'iléon isolé du cobaye selon la méthode suivante.

Des segments d'iléon de cobayes de 2,5 - 3 cm ont été lavés et immédiatement suspendus dans un bain contenant 10 ml d'une solution de Tyrode à 37° C et aérée avec un mélange d'oxygène (95%) et de gaz carbonique (5%). Après une période de stabilisation de 30 minutes au moins, on enregistre les contractions, en maintenant la préparation sous tension constante de 1 g, à l'aide d'un capteur relié à un polygraphe. On a évalué l'action agoniste en laissant le composé en contact avec le tissu isolé pendant une période nécessaire à exprimer la contraction maximale ; ensuite, on a lavé avec la solution de Tyrode. On n'a ajouté la dose suivante au bain qu'après que la préparation fut revenue sur sa ligne de base. Comme produit de référence, on a employé l'arécoline. On a évalué l'action antagoniste sur les contractions induites par l'acétylcholine (1 x $10^{-6}$M), l'histamine (1 x $10^{-5}$M) et le chlorure de baryum (2 x $10^{-4}$M). L'atropine, le diphénydramine et la papavérine ont été employés comme produits de référence. Le temps de contact avant d'ajouter l'agoniste a été d'une minute.

Pour chaque composé les courbes dose-réponse sont obtenues avec 4 à 6 concentrations différentes et 3 à 5 épreuves indépendantes. L'activité agoniste est exprimée par le $pD_2$ (logarithme négatif de la dose qui produit 50% de l'effet maximum). L'activité antagoniste est exprimée par la $CI_{50}$ (concentration inhibant 50% de la réponse maximale). (concentration inhibant 50% de la réponse maximale).

Les résultats obtenus avec les composés des exemples 1 à 6 sont reportés dans le tableau suivant :

TABLEAU 2

| Composé de l'exemple | Antagoniste à différents agents ($CI_{50}$:M) | | | Action agoniste $pD_2$ |
|---|---|---|---|---|
| | ACh | Histam. | $BaCl_2$ | |
| 1 | $5,7 \times 10^{-7}$ | $> 10^{-5}$ | $> 10^{-5}$ | < 5 |
| 2 | $3,4 \times 10^{-7}$ | $> 10^{-5}$ | $> 10^{-5}$ | < 5 |
| 3 | $2,6 \times 10^{-6}$ | | $> 10^{-5}$ | < 5 |
| 4 | $3,6 \times 10^{-6}$ | | $> 10^{-5}$ | < 5 |
| 5 | $2,8 \times 10^{-7}$ | | $> 10^{-5}$ | < 5 |
| 6 | $1,9 \times 10^{-7}$ | | $> 10^{-5}$ | < 5 |
| Atropine | $9,5 \times 10^{-9}$ | | | |
| Diphénydramine | | $8,3 \times 10^{-7}$ | | |
| Papavérine | | | $4,5 \times 10^{-5}$ | |
| Arécoline | | | | 6,68 |

Les études "in vitro" sur l'iléon isolé de cobaye ont mis en évidence que les composés de l'invention sont des agents antimuscariniques. Ils antagonisent les contractions induites par l'acétylcholine mais non celles induites par l'histamine et le chlorure de baryum.

3) **Action anticholinergique "in vivo".**

L'activité anticholinergique des composés a été déterminée en évaluant la capacité d'inhiber les effets cholinomimétiques induits par le carbachol. Le sulfate d'atropine a été employé comme produit de référence.

On utilise des souris mâles $CD_1$ pesant 25 à 30 g. Elles sont réparties en groupes de 6 animaux et traitées par voie intrapéritonéale à des doses scalaires des produits ou 0,25% de Méthocel pour les témoins. On utilise 12 animaux pour chaque dose. 30 minutes après l'administration des composés, on injecte aux souris par voie sous-cutanée 1 mg/kg de carbachol, dissous dans du sérum physiologique.

Chaque animal a été examiné 30 minutes après l'injection de carbachol pour évaluer la présence de diarrhée, salivation et larmoiement ; on a mesuré aussi la température corporelle au moyen d'un thermocouple inséré de 1,5 cm dans le rectum.

Le carbachol (1 mg/kg s.c.) a induit diarrhée, salivation et larmoiement chez toutes les souris témoins et une diminution de la température rectale de 2,5° C environ.

Pour chaque composé, nous avons déterminé et reporté dans le tableau suivant la dose capable d'inhiber chez 50% des animaux, l'apparition des symptomes cholinomimétiques induits par le carbachol et d'augmenter de 1° C l'effet hypothermique induit par l'agent cholinergique.

TABLEAU 3

| Composé de l'exemple | Dose mg/kg i.p. | | | Température corporelle |
|---|---|---|---|---|
| | Diarrhée | salivation | larmoiement | |
| 1 | 5 | > 50 | > 50 | > 50 |
| 2 | 8 | > 50 | > 50 | > 50 |
| 3 | 3 | > 50 | > 50 | > 50 |
| 4 | 7 | > 50 | > 50 | > 50 |
| 5 | 0,6 | 15 | > 50 | 12 |
| 6 | 2 | 40 | > 50 | > 50 |
| Atropine | 0,04 | 0,06 | 0,05 | 0,03 |

Les résultats obtenus montrent que, contrairement à l'atropine, les composés exercent "in vivo" une action anticholinergique sélective au niveau de la musculature intestinale.

## Revendications

1.- Les composés de formule (I) :

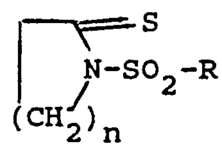

(I)

dans laquelle
- ou bien R représente un radical

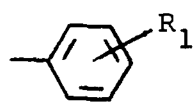

dans lequel $R_1$ en position quelconque sur le noyau phényle représente
soit un radical alcoyle linéaire, ramifié ou cyclique, saturé ou insaturé, renfermant jusqu'à 8 atomes de carbone,
soit un radical

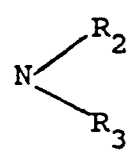

dans lequel $R_2$ et $R_3$ identiques ou différents représentent un atome d'hydrogène, un radical alcoyle linéaire, saturé ou insaturé renfermant jusqu'à 8 atomes de carbone, ou forment ensemble avec l'atome d'azote auquel ils sont liés un radical hétérocyclique carboné renfermant éventuellement un autre hétéroatome,
soit un radical $OR'$, $R'$ représentant un atome d'hydrogène, un radical alcoyle linéaire, ramifié ou cyclique renfermant jusqu'à 8 atomes de carbone, ou un radical aryle renfermant jusqu'à 14 atomes de carbone,
soit un radical $SR_4$ ou $S(O)R_5$, $R_4$ et $R_5$ représentant un radical alcoyle linéaire, ramifié ou cyclique, saturé ou insaturé renfermant jusqu'à 8 atomes de carbone,
- ou bien R représente un radical naphtyle, éventuellement substitué par un radical $R'_1$, $R'_1$ pouvant prendre l'une des valeurs indiquées ci-dessus pour $R_1$ et n représente le nombre 1 ou le nombre 2.

2.- Les composés de formule (I) tels que définis à la revendication 1, dans lesquels R représente un radical

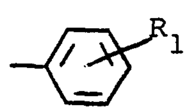

$R_1$ conservant la même signification que dans la revendication 1.

3.- Les composés de formule (I) tels que définis à la revendication 2, dans lesquels le radical $R_1$ est en position 4.

4.- Les composés de formule (I) tels que définis à la revendication 2 ou 3, dans lesquels $R_1$ représente un radical alcoyle linéaire ou ramifié renfermant jusqu'à 8 atomes de carbone et notamment un radical tert-butyle.

5.- Les composés de formule (I) tels que définis à la revendication 2 ou 3, dans lesquels $R_1$ représente un radical

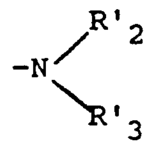

dans lequel $R'_2$ et $R'_3$ représentent un radical alcoyle linéaire ou ramifié renfermant jusqu'à 8 atomes de carbone ou forment avec l'atome d'azote auquel ils sont liés un radical hétérocyclique.

6.- Les composés de formule (I) tels que définis à la revendication 2 ou 3, dans lesquels $R_1$ représente un radical $SR_4$, $R_4$ représentant un radical alcoyle linéaire ou ramifié renfermant jusqu'à 4 atomes de carbone et notamment un radical méthyle.

7.- Les composés de formule (I) tels que définis à la revendication 1, dont les noms suivent :
- la 1-[4-(diéthylamino) phénylsulfonyl] pyrrolidin-2-thione,
- la 1-[4-(diéthylamino) phénylsulfonyl] pipéridin-2-thione,
- la 1-[4-(1-pipéridinyl) phénylsulfonyl] pyrrolidin-2-thione,
- la 1-[4-(1-pipéridinyl) phénylsulfonyl] pipéridin-2-thione,
- la 1-[4-(1-hexahydroazépinyl) phénylsulfonyl] pyrrolidin-2-thione,
- la 1-[4-(1-hexahydroazépinyl) phénylsulfonyl] pipéridin-2-thione,

8.- Procédé de préparation des composés de formule (I), tels que définis à la revendication 1, caractérisé en ce que l'on soumet un composé de formule (II) :

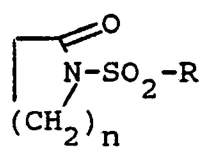

(II)

dans laquelle n et R conservent leur signification précédente à l'action d'un agent capable de convertir le radical carbonyle en radical thiocarbonyle pour obtenir le composé de formule (I) correspondant.

9.- Procédé de préparation des composés de formule (I) tels que définis à la revendication 1, caractérisé en ce que l'agent capable de convertir le radical carbonyle en radical thiocarbonyle est le réactif de LAWESSON de formule :

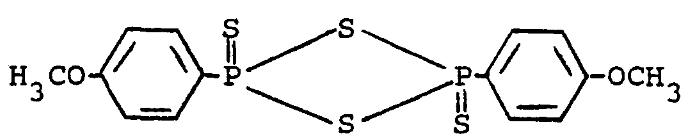

10.- A titre de médicaments, les composés de formule (I) tels que définis à l'une quelconque des revendications 1 à 6.

11.- A titre de médicament, l'un quelconque des composés de formule (I) tels que définis à la revendication 7.

12.- Les compositions pharmaceutiques renfermant au moins un médicament tel que défini à la revendication 10 ou 11.

Revendications pour l'Etat contractant suivant: ES

1.- Procédé de préparation des composés de formule (I) :

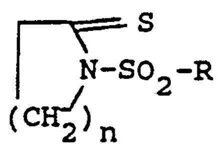

(I)

dans laquelle
- ou bien R représente un radical

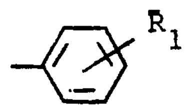

dans lequel $R_1$ en position quelconque sur le noyau phényle représente
soit un radical alcoyle linéaire, ramifié ou cyclique, saturé ou insaturé, renfermant jusqu'à 8 atomes de carbone,
soit un radical

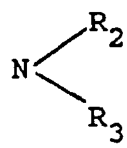

dans lequel $R_2$ et $R_3$ identiques ou différents représentent un atome d'hydrogène, un radical alcoyle linéaire, saturé ou insaturé renfermant jusqu'à 8 atomes de carbone, ou forment ensemble avec l'atome d'azote auquel ils sont liés un radical hétérocyclique carboné renfermant éventuellement un autre hétéroatome,
soit un radical $OR'$, $R'$ représentant un atome d'hydrogène, un radical alcoyle linéaire, ramifié ou cyclique renfermant jusqu'à 8 atomes de carbone, ou un radical aryle renfermant jusqu'à 14 atomes de carbone,
soit un radical $SR_4$ ou $S(O)R_5$, $R_4$ et $R_5$ représentant un radical alcoyle linéaire, ramifié ou cyclique, saturé ou insaturé renfermant jusqu'à 8 atomes de carbone,
- ou bien R représente un radical naphtyle, éventuellement substitué par un radical $R'_1$, $R'_1$ pouvant prendre l'une des valeurs indiquées ci-dessus pour $R_1$ et n représente le nombre 1 ou le nombre 2, caractérisé en ce que l'on soumet un composé de formule (II) :

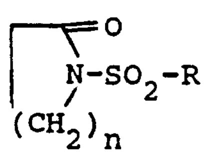

(II)

dans laquelle n et R conservent leur signification précédente à l'action d'un agent capable de convertir le radical carbonyle en radical thiocarbonyle pour obtenir le composé de formule (I) correspondant.

2.- Procédé selon la revendication 1, caractérisé en ce que l'on utilise au départ un produit de formule (II) dans laquelle R représente un radical

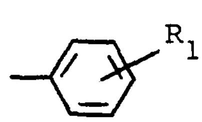

$R_1$ conservant la même signification que dans la revendication 1.

3.- Procédé selon la revendication 2, caractérisé en ce que le radical $R_1$ est en position 4.

4.- Procédé selon la revendication 2 ou 3, caractérisé en ce que $R_1$ représente un radical alcoyle linéaire ou ramifié renfermant jusqu'à 8 atomes de carbone et notamment un radical tert-butyle.

5.- Procédé selon la revendication 2 ou 3, caractérisé en ce que $R_1$ représente un radical

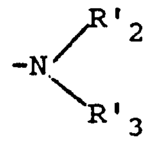

dans lequel $R'_2$ et $R'_3$ représentent un radical alcoyle linéaire ou ramifié renfermant jusqu'à 8 atomes de carbone ou forment avec l'atome d'azote auquel ils sont liés un radical hétérocyclique.

6.- Procédé selon la revendication 2 ou 3, caractérisé en ce que $R_1$ représente un radical $SR_4$, $R_4$ représentant un radical alcoyle linéaire ou ramifié renfermant jusqu'à 4 atomes de carbone et notamment un radical méthyle.

7.- Procédé selon la revendication 1, caractérisé en ce que l'on choisit le produit de formule (II) de manière telle que l'on prépare l'un des composés dont les noms suivent :
- la 1-[4-(diéthylamino) phénylsulfonyl] pyrrolidin-2-thione,
- la 1-[4-(diéthylamino) phénylsulfonyl] pipéridin-2-thione,
- la 1-[4-(1-pipéridinyl) phénylsulfonyl] pyrrolidin-2-thione,
- la 1-[4-(1-pipéridinyl) phénylsulfonyl] pipéridin-2-thione,
- la 1-[4-(1-hexahydroazépinyl) phénylsulfonyl] pyrrolidin-2-thione,
- la 1-[4-(1-hexahydroazépinyl) phénylsulfonyl] pipéridin-2-thione.

Revendications pour l'Etat contractant suivant: GR

1.- Procédé de préparation des composés de formule (I) :

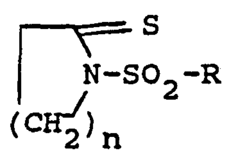

(I)

dans laquelle
- ou bien R représente un radical

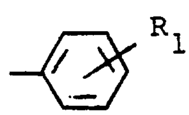

dans lequel $R_1$ en position quelconque sur le noyau phényle représente
soit un radical alcoyle linéaire, ramifié ou cyclique, saturé ou insaturé, renfermant jusqu'à 8 atomes de carbone,
soit un radical

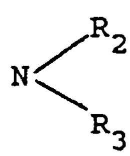

dans lequel $R_2$ et $R_3$ identiques ou différents représentent un atome d'hydrogène, un radical alcoyle linéaire, saturé ou insaturé renfermant jusqu'à 8 atomes de carbone, ou forment ensemble avec l'atome d'azote auquel ils sont liés un radical hétérocyclique carboné renfermant éventuellement un autre hétéroatome,
soit un radical $OR'$, $R'$ représentant un atome d'hydrogène, un radical alcoyle linéaire, ramifié ou cyclique renfermant jusqu'à 8 atomes de carbone, ou un radical aryle renfermant jusqu'à 14 atomes de carbone,
soit un radical $SR_4$ ou $S(O)R_5$, $R_4$ et $R_5$ représentant un radical alcoyle linéaire, ramifié ou cyclique, saturé ou insaturé renfermant jusqu'à 8 atomes de carbone,
- ou bien R représente un radical naphtyle, éventuellement substitué par un radical $R'_1$, $R'_1$ pouvant prendre l'une des valeurs indiquées ci-dessus pour $R_1$ et n représente le nombre 1 ou le nombre 2,

caractérisé en ce que l'on soumet un composé de formule (II) :

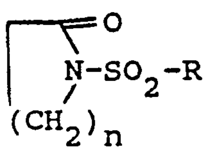

(II)

dans laquelle n et R conservent leur signification précédente à l'action d'un agent capable de convertir le radical carbonyle en radical thiocarbonyle pour obtenir le composé de formule (I) correspondant.

2.- Procédé selon la revendication 1, caractérisé en ce que l'on utilise au départ un produit de formule (II) dans laquelle R représente un radical

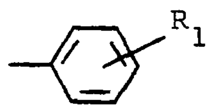

$R_1$ conservant la même signification que dans la revendication 1.

3.- Procédé selon la revendication 2, caractérisé en ce que le radical $R_1$ est en position 4.

4.- Procédé selon la revendication 2 ou 3, caractérisé en ce que $R_1$ représente un radical alcoyle linéaire ou ramifié renfermant jusqu'à 8 atomes de carbone et notamment un radical tert-butyle.

5.- Procédé selon la revendication 2 ou 3, caractérisé en ce que $R_1$ représente un radical

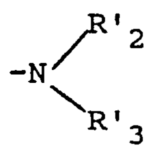

dans lequel $R'_2$ et $R'_3$ représentent un radical alcoyle linéaire ou ramifié renfermant jusqu'à 8 atomes de carbone ou forment avec l'atome d'azote auquel ils sont liés un radical hétérocyclique.

6.- Procédé selon la revendication 2 ou 3, caractérisé en ce que $R_1$ représente un radical $SR_4$, $R_4$ représentant un radical alcoyle linéaire ou ramifié renfermant jusqu'à 4 atomes de carbone et notamment un radical méthyle.

7.- Procédé selon la revendication 1, caractérisé en ce que l'on choisit le produit de formule (II) de manière telle que l'on prépare l'un des composés dont les noms suivent :
- la 1-[4-(diéthylamino) phénylsulfonyl] pyrrolidin-2-thione,
- la 1-[4-(diéthylamino) phénylsulfonyl] pipéridin-2-thione,
- la 1-[4-(1-pipéridinyl) phénylsulfonyl] pyrrolidin-2-thione,
- la 1-[4-(1-pipéridinyl) phénylsulfonyl] pipéridin-2-thione,
- la 1-[4-(1-hexahydroazépinyl) phénylsulfonyl] pyrrolidin-2-thione,
- la 1-[4-(1-hexahydroazépinyl) phénylsulfonyl] pipéridin-2-thione.

8.- Procédé de préparation des compositions pharmaceutiques, caractérisé en ce que l'on met à titre de principe actif, l'un au moins des composés de formule (I) telle que définie à la revendication l, sous une forme destinée à cet usage.

9.- Procédé selon la revendication 8, caractérisé en ce que l'on utilise à titre de principe actif l'un au moins des dérivés de formule (1) tels qu'obtenus à la revendication 7.